# EUROPÄISCHE PATENTSCHRIFT

(11) **EP 0 773 946 B1**
(45) Veröffentlichungstag und Bekanntmachung des Hinweises auf die Patenterteilung: **27.03.2002**
(21) Anmeldenummer: 95927727.8
(22) Anmeldetag: 26.07.1995
(51) Int. Cl.: C07D 471/04, A61K 31/435

(54) **IMIDAZOPYRIDIN-AZOLIDINONE**
IMIDAZOPYRIDINE-AZOLIDINONES
IMIDAZOPYRIDINAZOLIDINONES

(30) Priorität: 28.07.1994 CH 239194
(43) Veröffentlichungstag der Anmeldung: 21.05.1997
(73) Patentinhaber: Byk Gulden Lomberg Chemische Fabrik GmbH, 78467 Konstanz (DE)
(72) Erfinder: SIMON, Wolfgang-Alexander, D-78464 Konstanz (DE); RIEDEL, Richard, D-88339 Bad Waldsee (DE); POSTIUS, Stefan, D-78467 Konstanz (DE); KLEY, Hans-Peter, D-78476 Allensbach (DE); GRUNDLER, Gerhard, D-78464 Konstanz (DE); RAINER, Georg, D-78464 Konstanz (DE); SENN-BILFINGER, Jörg, D-78464 Konstanz (DE); THIBAUT, Ulrich, D-78462 Konstanz (DE)
(86) Internationale Anmeldenummer: EP9502954
(87) Internationale Veröffentlichungsnummer: WO9603405

(56) Entgegenhaltungen:
- EP-A- 0 268 989
- EP-A- 0 308 917
- EP-A- 0 596 406
- E. Mutschler, Arzneimittelwirkungen, Wissenschaftliche Verlagsges.mbH Stuttgart, 1996, Seiten 399-400

## Beschreibung

### Anwendungsgebiet der Erfindung

Die Erfindung betrifft neue Imidazopyridin-Azolidinone, die in der pharmazeutischen Industrie als Wirkstoffe für die Herstellung von Arzneimitteln verwendet werden sollen.

### Bekannter technischer Hintergrund

In der europäischen Patentanmeldung EP-A-0 033 094 werden Imidazo[1,2-a]pyridine beschrieben, die in 8-Position einen Arylsubstituenten tragen, der bevorzugt ein Phenyl-, Thienyl-, Pyridyl- oder ein durch Chlor, Fluor, Methyl, tert.-Butyl, Trifluormethyl, Methoxy oder Cyan substituierter Phenylrest ist. Als besonders interessante Arylreste sind in der EP-A-0 033 094 die Reste Phenyl, o- oder p-Fluorphenyl, p-Chlorphenyl und 2,4,6-Trimethylphenyl genannt, wovon die Reste Phenyl, o- oder p-Fluorphenyl und 2,4,6-Trimethylphenyl besonders bevorzugt sind. - In den europäischen Patentanmeldungen EP-A-0 204 285, EP-A-0 228 006, EP-A-0 268 989 und EP-A-0 308 917 werden Imidazo[1,2-a]pyridine beschrieben, die in 3-Position einen ungesättigten aliphatischen Rest, insbesondere einen (substituierten) Alkinylrest tragen. - In der europäischen Patentanmeldung EP-A-0 266 890 werden Imidazo[1,2-a]pyridine beschrieben, die in 8-Position durch einen Alkenyl-, Alkyl- oder Cycloalkylalkylrest substituiert sind.

Aus der europäischen Patentanmeldung EP-A-0 596 406 sind 3-Halogen-imidazo[1,2-a]pyridine bekannt, die in 8-Position eine Arylalkyloxy- bzw. Arylalkylaminogruppe tragen, die ihrerseits unter anderem durch eine mit Oxo substituierte heterocyclische Gruppe substituiert sein kann.

### Beschreibung der Erfindung

Es wurde nun gefunden, daß die nachfolgend näher beschriebenen Verbindungen, die sich von den Verbindungen des Standes der Technik insbesondere durch die Substitution in 8-Position unterscheiden, überraschende und besonders vorteilhafte Eigenschaften besitzen.

Gegenstand der Erfindungen sind Verbindungen der Formel I (siehe beigefügtes Formelblatt I), worin
- R: 1-4C-Alkyl, Hydroxymethyl, Halogen oder Thiocyanat,
- R1: 1-4C-Alkyl,
- R2: Wasserstoff, 1-4C-Alkyl, 1-4C-Alkoxy, Halogen oder Trifluormethyl,
- R3: Wasserstoff oder 1-4C-Alkyl,
- R3': Wasserstoff, 1-4C-Alkyl oder substituiertes 1-4C-Alkyl mit einem oder zwei gleichen oder verschiedenen Substituenten ausgewählt aus der Gruppe bestehend aus Halogen, 1-4C-Alkoxy oder 1-4C-Alkoxy-1-4C-alkoxy,
- R4: Wasserstoff, 1-4C-Alkyl, 1-4C-Alkoxy, Halogen oder Trifluormethyl,
- R5: Wasserstoff, 1-4C-Alkyl, 1-4C-Alkoxy oder Halogen,
- A: 0 (Sauerstoff) oder NH und
- Y: 0 (Sauerstoff) oder CH₂ bedeutet,
und ihre Salze.

1-4C-Alkyl steht für geradkettige oder verzweigte Alkylreste mit 1 bis 4 Kohlenstoffatomen. Beispielsweise seien genannt der Butyl-, iso-Butyl-, sec.-Butyl-, tert.-Butyl-, Propyl-, Isopropyl-, Ethyl- und insbesondere der Methylrest.

Halogen im Sinne der Erfindung ist Brom, Fluor und insbesondere Chlor.

1-4C-Alkoxy steht für ein Sauerstoffatom, an das einer der vorstehend genannten 1-4C-Alkylreste gebunden ist. Beispielsweise sei der Methoxy- und der Ethoxyrest genannt.

1-4C-Alkoxy-1-4C-alkoxy steht für einen der vorstehend genannten 1-4C-Alkoxyreste, der durch einen weiteren 1-4C-Alkoxyrest substituiert ist. Beispielsweise sei der Methoxyethoxyrest genannt.

Als Salze kommen für Verbindungen der Formel I bevorzugt alle Säureadditionssalze in Betracht. Besonders erwähnt seien die pharmakologisch verträglichen Salze der in der Galenik üblicherweise verwendeten anorganischen und organischen Säuren. Pharmakologisch unverträgliche Salze, die beispielsweise bei der Herstellung der erfindungsgemäßen Verbindungen im industriellen Maßstab als Verfahrensprodukte zunächst anfallen können, werden durch dem Fachmann bekannte Verfahren in pharmakologisch verträgliche Salze übergeführt. Als solche eignen sich wasserlösliche und wasserunlösliche Säureadditionssalze mit Säuren wie beispielsweise Salzsäure, Bromwasserstoffsäure, Phosphorsäure, Salpetersäure, Schwefelsäure, Essigsäure, Zitronensäure, D-Gluconsäure, Benzoesäure, 2-(4-Hydroxybenzoyl)-benzoesäure, Buttersäure, Sulfosalicylsäure, Maleinsäure, Laurinsäure, Äpfelsäure, Fumarsäure, Bernsteinsäure, Oxalsäure, Weinsäure, Embonsäure, Stearinsäure, Toluolsulfonsäure, Methansulfonsäure oder 3-Hydroxy-2-naphthoesäure, wobei die Säuren bei der Salzherstellung - je nachdem, ob es sich um eine ein- oder mehrbasige Säure handelt und je nachdem, welches Salz gewünscht wird - im äquimolaren oder einem davon abweichenden Mengenverhältnis eingesetzt werden.

Hervorzuhebende Verbindungen sind solche der Formel I, worin
- R: 1-4C-Alkyl, Hydroxymethyl oder Halogen,
- R1: 1-4C-Alkyl,
- R2: 1-4C-Alkyl oder Halogen,
- R3: Wasserstoff oder 1-4C-Alkyl,
- R3': Wasserstoff, 1-4C-Alkyl oder substituiertes 1-4C-Alkyl mit einem Substituenten ausgewählt aus der Gruppe bestehend aus Halogen, 1-4C-Alkoxy und 1-4C-Alkoxy-1-4C-alkoxy,
- R4: Wasserstoff,
- R5: Wasserstoff,
- A: 0 (Sauerstoff) oder NH und
- Y: 0 (Sauerstoff) oder CH₂ bedeutet,
und ihre Salze.

Besonders hervorzuhebende Verbindungen sind solche der Formel I, worin
- R: Methyl, Hydroxymethyl,Chlor oder Fluor,
- R1: Methyl,
- R2: 1-4C-Alkyl,
- R3: Wasserstoff oder 1-4C-Alkyl,
- R3': Wasserstoff, 1-4C-Alkyl oder substituiertes 1-4C-Alkyl mit einem Substituenten ausgewählt aus der Gruppe bestehend aus 1-4C-Alkoxy und 1-4C-Alkoxy-1-4C-alkoxy,
- R4: Wasserstoff,
- R5: Wasserstoff,
- A: 0 (Sauerstoff) oder NH und
- Y: 0 (Sauerstoff) oder CH₂ bedeutet,
und ihre Salze.

Bevorzugte Verbindungen sind solche der Formel I, worin
- R: Methyl, Hydroxymethyl oder Chlor,
- R1: Methyl,
- R2: 1-4C-Alkyl,
- R3: Wasserstoff oder 1-4C-Alkyl,
- R3': Wasserstoff, 1-4C-Alkyl, 1-4C-Alkoxy-1-4C-alkyl oder 1-4C-Alkoxy-1-4C- alkoxy-1-4C-alkyl,
- R4: Wasserstoff,
- R5: Wasserstoff,
- A: 0 (Sauerstoff) oder NH
- Y: 0 (Sauerstoff) oder CH₂ bedeutet,
und ihre Salze.

Beispielhafte erfindungsgemäße Verbindungen sind in der folgenden Tabelle 1 aufgeführt:

**Tabelle 1**

| Verbindungen der Formel I (siehe beigefügtes Formelblatt) mit R1=CH₃, R4=H, R5=H und den folgenden weiteren Substituenten- und Symbolbedeutungen: | | | | | |
|---|---|---|---|---|---|
| R | R2 | R3 | R3' | A | Y |
| F | CH₃ | H | H | NH | 0 |
| F | CH₃ | H | H | NH | CH₂ |
| F | CH₃ | H | H | 0 | 0 |
| F | CH₃ | H | H | 0 | CH₂ |
| Cl | CH₃ | H | H | 0 | 0 |
| Cl | CH₃ | H | H | NH | CH₂ |
| Cl | CH₃ | H | H | 0 | CH₂ |
| CH₃ | Cl | H | H | NH | 0 |
| CH₂OH | Cl | H | H | NH | 0 |
| CH₃ | Cl | H | H | 0 | 0 |
| CH₃ | Cl | H | H | NH | CH₂ |
| Cl | Cl | H | H | NH | CH₂ |
| CH₂OH | CH₃ | H | H | NH | CH₂ |
| CH₃ | CH₃ | H | CH₂OCH₂CH₂OCH₃ | NH | 0 |
| CH₃ | Cl | H | CH₂OCH₃ | NH | 0 |

und die Salze der in der Tabelle genannten Verbindungen.

Verbindungen der Formel I können an den Positionen, an denen die Substituenten R3 bzw. R3' angebunden sind, jeweils ein Chiralitätszentrum haben. Die Erfindung umfaßt bei den chiralen Verbindungen daher sowohl die reinen Enantiomeren und Diastereomeren als auch deren Gemische in jedem Mischungsverhältnis, einschließlich der Racemate.

Das Verfahren zur Herstellung der Verbindungen der Formel I und ihrer Salze ist dadurch gekennzeichnet, daß man
a) zur Herstellung von Verbindungen der Formel I, in denen R Hydroxymethyl bedeutet, Verbindungen der Formel II (siehe beigefügtes Formelblatt I), worin R1, R2, R3, R3', R4, R5, A und Y die oben angegebenen Bedeutungen haben, reduziert, oder daß man
b) Verbindungen der Formel III (siehe beigefügtes Formelblatt II), worin R , R1, R2, R3, R3', R4, R5, A und Y die oben angegebenen Bedeutungen haben und X eine geeignete Abgangsgruppe darstellt, unter HX-Abspaltung cyclisiert,
und daß man gewünschtenfalls anschließend die erhaltenen Verbindungen I in ihre Salze überführt, oder daß man gewünschtenfalls anschließend aus erhaltenen Salzen der Verbindungen I die Verbindungen I freisetzt.

Die Reduktion der Verbindungen II wird in einer dem Fachmann an sich gewohnten Weise vorgenommen. Sie erfolgt in inerten Lösungsmitteln, z.B. niederen aliphatischen Alkoholen, z.B. unter Verwendung geeigneter Hydride, wie beispielsweise Natriumborhydrid, gewünschtenfalls unter Zusatz von Wasser.

Die Cyclisierung der Verbindungen III erfolgt auf eine dem Fachmann an sich vertraute Weise, beispielsweise so wie in den nachfolgenden Beispielen beschrieben, in inerten Lösungsmitteln und in Gegenwart eines säurebindenden Mittels (Protonenakzeptors). Als Protonenakzeptoren seien beispielsweise Alkalihydroxide, wie Natrium- oder Kaliumhydroxid, oder Metallhydride, wie Natriumhydrid genannt. Eine geeignete Abgangsgruppe ist beispielsweise ein Halogenatom (bevorzugt Chlor oder Brom) oder eine Methansulfonyloxygruppe.

Welche Reaktionsbedingungen für die Durchführung des Verfahrens im einzelnen erforderlich sind, ist dem Fachmann aufgrund seines Fachwissens geläufig.

Die Isolierung und Reinigung der erfindungsgemäßen Substanzen erfolgt in an sich bekannter Weise z.B. derart, daß man das Lösungsmittel im Vakuum abdestilliert und den erhaltenen Rückstand aus einem geeigneten Lösungsmittel umkristallisiert oder einer der üblichen Reinigungsmethoden, wie beispielsweise der Säulenchromatographie an geeignetem Trägermaterial, unterwirft.

Säureadditionssalze erhält man durch Auflösen der freien Base in einem geeigneten Lösungsmittel, z.B. in Wasser, in einem chlorierten Kohlenwasserstoff, wie Methylenchlorid oder Chloroform, einem niederen aliphatischen Alkohol (Ethanol, Isopropanol), einem Keton, wie Aceton, oder einem Ether, wie THF oder Diisopropylether, das die gewünschte Säure enthält, oder dem die gewünschte Säure anschließend zugegeben wird.

Die Salze werden durch Filtrieren, Umfällen, Ausfällen mit einem Nichtlösungsmittel für das Anlagerungssalz oder durch Verdampfen des Lösungsmittels gewonnen. Erhaltene Salze können durch Alkalisierung, z.B. mit wäßriger Ammoniaklösung, in die freien Basen umgewandelt werden, welche wiederum in Säureadditionssalze übergeführt werden können. Auf diese Weise lassen sich pharmakologisch nicht verträgliche Säureadditionssalze in pharmakologisch verträgliche Säureadditionssalze umwandeln.

Die Ausgangsverbindungen II werden durch Cyclisierung solcher Verbindungen III erhalten, in denen R die Bedeutung CHO hat.

Die Verbindungen III werden beispielsweise durch Umsetzung von Verbindungen IV (siehe beigefügtes Formelblatt II), worin R , R1, R2, R4, R5 und A die oben angegebenen Bedeutungen haben, mit Verbindungen Hal-CO-Y-CHR3'-CHR3-X, worin R3, R3', Y und X die oben angegebenen Bedeutungen haben und Hal ein Halogenatom (bevorzugt Chlor oder Brom) darstellt, erhalten.

Die Ausgangsverbindungen IV sind aus den EP-A-0 268 989 und EP-A-0 308 917 bekannt bzw. sie können auf analoge Weise wie dort beschrieben hergestellt werden. Beispielsweise können die Ausgangsverbindungen IV in an sich bekannter Weise aus den entsprechenden Nitroverbindungen durch Reduktion oder durch Hydrolyse geeigneter N-Acylderivate hergestellt werden.

Die folgenden Beispiele dienen der näheren Erläuterung zur Herstellung der erfindungsgemäßen Verbindungen. Insbesondere dienen die Beispiele auch dazu, die Herstellung der Verbindungen der Formel I sowie die Herstellung ausgewählter Ausgangsverbindungen exemplarisch zu beschreiben. Ebenso können weitere Verbindungen der Formel I sowie weitere Ausgangsverbindungen, deren Herstellung nicht explizit beschrieben ist, in analoger oder in einer dem Fachmann an sich vertrauten Weise unter Anwendung üblicher Verfahrenstechniken hergestellt werden. Die Abkürzung RT steht für Raumtemperatur, h steht für Stunde(n), min für Minute(n), Schmp. für Schmelzpunkt, Kp. für Siedepunkt, Zers. für Zersetzung.

### Beispiele

### Endprodukte

### 1. 3-[2-(2,3-dimethyl-imidazo[1,2-a]pyridin-8-ylaminomethyl)-3-methylphenyl]-oxazolidin-2-on

### a) 8-[2-(2-Chlorethoxycarbonylamino)-6-methyl-benzylamino]-2,3-dimethylimidazo[1,2-a]pyridin

Zu einer Lösung von 8-(2-Amino-6-methyl-benzylamino)-2,3-dimethyl-imidazo[1,2-a]pyridin (19,65 g, 70 mmol) in wasserfreiem Dichlormethan (600 ml) wird bei RT, während ca. 30 min, Chlorameisensäure-2-chlorethylester (12,4 g, 81 mmol) gelöst in Dichlormethan (40 ml) getropft. Dann wird 16 h bei RT gerührt und anschließend mit Natriumhydrogencarbonatlösung (4 x 200 ml) extrahiert. Nach Extraktion der wäßrigen Phasen mit Dichlormethan (200 ml) werden die vereinigten organischen Extrakte mit Wasser (2 x 200 ml) gewaschen, über Magnesiumsulfat getrocknet und eingeengt. Das verbleibende Rohprodukt (33,9 g) wird direkt für die weitere Umsetzung in Beispiel 1b verwendet.

### b) 3-[2-(2,3-dimethyl-imidazo[1,2-a]pyridin-8-ylaminomethyl)-3-methylphenyl]-oxazolidin-2-on

Zu einer Lösung des Rohproduktes aus Beispiel 1a (33,8 g) in wasserfreiem Ethanol (800 ml) gibt man unter starkem Rühren portionsweise während ca. 30 min Natriumhydrid (3,15 g, 80 %ig in Paraffin). Man rührt danach noch 30 min bei RT, gibt anschließend portionsweise Wasser (200 ml) zu und destilliert das Ethanol am Rotationsverdampfer ab. Der Niederschlag wird abgesaugt, mit Wasser gewaschen und im Vakuum getrocknet. Das Rohprodukt wird durch Chromatographie an Kieselgel (Fließmittel: Toluol/Dioxan = 4:1) gereinigt. Nach Einengen der Fraktionen mit Rf = 0,15 und Kristallisation aus Essigester/ Diisopropylether wird die Titelverbindung (13,6 g, 63 %) als beiger Feststoff isoliert. Schmp. 139-140°C.

### c) Durch Umsetzung der in Aceton gelösten Titelverbindung mit Methansulfonsäure erhält man das Methansulfonat der Titelverbindung vom Schmp. 232-235°C.

### 2. 3-[2-(3-Chlor-2-methyl-imidazo[1,2-a]pyridin-8-ylaminomethyl)-3-methylphenyl]-oxazolidin-2-on

### a) 8-[2-(2-Chlorethoxycarbonylamino)-6-methyl-benzylamino]-3-chlor-2-methyl-imidazo[1,2-a]pyridin

Nach der in Beispiel 1a angegebenen Arbeitsweise erhält man aus 8-(2-Amino-6-methyl-benzylamino)-3-chlor-2-methyl-imidazo[1,2-a]pyridin (0,27 g) und Chlorameisensäure-2-chlorethylester (0,18 g) in Dichlormethan (30 ml) die Titelverbindung als braunes Öl, das direkt für die weitere Umsetzung in 2b verwendet wird.

### b) 3-[2-(3-Chlor-2-methyl-imidazo[1,2-a]pyridin-8-ylaminomethyl)-3-methylphenyl]-oxazolidin-2-on

Nach der in Beispiel 1b angegebenen Arbeitsweise erhält man aus dem Rohprodukt 2a (0,37 g) und Natriumhydrid (0,45 g, 80 %ig in Paraffin) in wasserfreiem Ethanol (20 ml) nach Kristallisation aus Essigester/Petrolether die Titelverbindung (70 mg, 21 %) als beigen Feststoff. Schmp. 137-139°C.

### 3. 3-[2-(3-Hydroxymethyl-2-methyl-imidazo[1,2-a]pyridin-8-ylaminomethyl)-3-methyl-phenyl]-oxazolidin-2-on

### a) 8-[2-(2-Chlorethoxycarbonylamino)-6-methyl-benzylamino]-3-formyl-2-methyl-imidazo[1,2-a]pyridin

Nach der in Beispiel 1a angegebenen Arbeitsweise erhält man aus 8-(2-Amino-6-methyl-benzylamino)-3-formyl-2-methyl-imidazo[1,2-a]pyridin (2,03 g) und Chlorameisensäure-2-chlorethylester (1,01 g) in Dichlormethan (120 ml) die Titelverbindung als braunes Öl, das direkt für die weitere Umsetzung in Beispiel 3b verwendet wird.

### b) 3-[2-(3-Formyl-2-methyl-imidazo[1,2-a]pyridin-8-ylaminomethyl)-3-methyl-phenyl]-oxazolidin-2-on

Nach der in Beispiel 1b angegebenen Arbeitsweise erhält man aus dem Rohprodukt 3a (0,12 g) und Natriumhydrid (0,01 g, 80 %ig in Paraffin) in wasserfreiem Ethanol (10 ml) nach Kristallisation aus Essigester/Diisopropylether die Titelverbindung (80 mg, 73 %) als beigen Feststoff. Schmp. 196-197°C.

### c) 3-[2-(3-Hydroxymethyl-2-methyl-imidazo[1,2-a]pyridin-8-ylaminomethyl)-3-methyl-phenyl]-oxazolidin-2-on

Eine Lösung von 3-[2-(3-Formyl-2-methyl-imidazo[1,2-a]pyridin-8-ylaminomethyl)-3-methyl-phenyl]-oxazolidin-2-on (0,18 g, 0,49 mmol) und Natriumboranat (19 mg, 0,5 mmol) in wasserfreiem Ethanol (20 ml) wird 30 min bei RT gerührt. Dann wird Wasser (75 ml) zugegeben, das Ethanol am Rotationsverdampfer abdestilliert und der wäßrige Rückstand wird mit Essigester (3 x 50 ml) extrahiert. Die organischen Extrakte werden mit Wasser (50 ml) gewaschen, über Magnesiumsulfat getrocknet und eingeengt. Nach Kristallisation aus Diisopropylether erhält man die Titelverbindung (120 mg, 66 %) als beigen Feststoff. Schmp. 152-157°C.

### 4. 3-[2-(3-Hydroxymethyl-2-methyl-imidazo[1,2-a]pyridin-8-yloxymethyl)-3-methyl-phenyl]-oxazolidin-2-on

### a) 8-[2-(2-Chlorethoxycarbonylamino)-6-methyl-benzyloxy]-3-formyl-2-methyl-imidazo[1,2-a]pyridin

Nach der in Beispiel 1a angegebenen Arbeitsweise erhält man aus 8-(2-Amino-6-methyl-benzyloxy)-3-formyl-2-methyl-imidazo[1,2-a]pyridin (2,36 g) und Chlorameisensäure-2-chlorethylester (1,3 g) in Dichlormethan (150 ml) die Titelverbindung als braunes Öl, das direkt für die weitere Umsetzung in 3b verwendet wird.

### b) 3-[2-(3-Formyl-2-methyl-imidazo[1,2-a]pyridin-8-yloxymethyl)-3-methylphenyl]-oxazolidin-2-on

Nach der in Beispiel 1b angegebenen Arbeitsweise erhält man aus dem Rohprodukt 4a (1,14 g) und Natriumhydrid (0,13 g, 80 %ig in Paraffin) in wasserfreiem Ethanol (200 ml) nach Kristallisation aus Essigester/Diisopropylether die Titelverbindung (0,7 g, 67 %) als beigen Feststoff. Schmp. 242-244°C.

### c) 3-[2-(3-Hydroxymethyl-2-methyl-imidazo[1,2-a]pyridin-8-yloxymethyl)-3-methyl-phenyl]-oxazolidin-2-on

Eine Lösung von 3-[2-(3-Formyl-2-methyl-imidazo[1,2-a]pyridin-8-yloxymethyl)-3-methyl-phenyl]-oxazolidin-2-on (1,39 g, 3,8 mmol) und Natriumboranat (0,19 g, 4,75 mmol) in wasserfreiem Ethanol (125 ml) wird 1,5 h bei RT gerührt. Dann wird Wasser (100 ml) zugegeben, das Ethanol am Rotationsverdampfer abdestilliert und der wäßrige Rückstand 30 min bei 4°C gerührt. Der Miederschlag wird abfiltriert, mit kaltem Wasser gewaschen und im Vakuum getrocknet. Die Titelverbindung (1,28 g, 91 %) als beigen Feststoff. Schmp. 190-193°C.

### 5. 5-Chlormethyl-3-[2-(2,3-dimethyl-imidazo[1,2-a]pyridin-8-ylaminomethyl)-3-methyl-phenyl]-oxazolidin-2-on

a) Zu 591 ml (1,14 mol) einer Lösung von Phosgen in Toluol (1,93 mol/l) tropft man in 2 h bei 5-8°C eine Mischung von 150 g (1,14 mol) 1,3-Dichlorpropanol und 90,2 g (1,14 mol) Pyridin und rührt noch 24 h bei 20°C. Man begast mit N₂, filtriert Pyridinhydrochlorid ab, wäscht mit Wasser, trocknet mit Magnesiumsulfat und erhält nach fraktionierter Destillation 102 g (47 %) Chlorameisensäure-(2-chlor-1-chlormethylethylester). Kp. 140-145°C/18 mbar.

b) Man rührt eine Lösung von 2,0 g (7,13 mMol) 8-(2-Amino-6-methyl-benzylamino)-2,3-dimethyl-imidazo[1,2-a]pyridin und 1,5 g (7,84 mmol) der voranstehenden Verbindung in 80 ml Dichlormethan 2 Tage bei RT. Die organische Lösung wird mit gesättigter Natriumcarbonatlösung und Wasser gewaschen, mit Magnesiumsulfat getrocknet und im Vakuum eingeengt. Man erhält 3,4 g rohes 8-(2-[(2-Chlor-1-chlormethylethoxy)carbonylamino]-6-methyl-benzylamino)-2,3-dimethyl-imidazo(1,2-a]pyridin als Öl, welches in die nächste Stufe eingesetzt wird.

c) Zu einer Lösung der voranstehenden Verbindung in 30 ml Ethanol tropft man bei 20°C eine Lösung von 0,48 g 85 % Kaliumhydroxid in 4 ml Ethanol und rührt noch 5 h nach. Man konzentriert im Vakuum, versetzt mit Wasser, extrahiert mit Dichlormethan und engt die mit Magnesiumsulfat getrocknete organische Schicht im Vakuum ein. Man versetzt den Rückstand mit 5 ml Ethanol und kühlt im Eisbad. Man erhält 1,2 g der Titelverbindung. Schmp. 141-143°C.

### 6. 3-[2-(2,3-Dimethyl-imidazo[1,2-a]pyridin-8-ylaminomethyl)-3-methylphenyl]-5-methyl-oxazolidin-2-on-hydrochlorid

a) Analog Beispiel 5a erhält man aus 2-Chlor-1-propanol und Phosgen Chlorameisensäure-(2-chlor-1-methylethylester). Kp. 55-56°C/17 mbar.

b) Analog Beispiel 5b setzt man 2,0 g (7,13 mmol) 8-(2-Amino-6-methylbenzylamino)-2,3-dimethyl-imidazo[1,2-a]pyridin und 1,23 g (7,85 mmol) der voranstehenden Verbindung um und erhält 3,1 g rohes 8-{2-[(2-Chlor-1-methylethoxy)carbonylamino]-6-methyl-benzylamino}-2,3-dimethyl-imidazo[1,2-a]pyridin als amorphe Masse.

c) Man tropft zu 2,9 g der voranstehenden Verbindung in 15 ml Ethanol bei RT eine Lösung von 0,48 g 85 % KOH in 15 ml Ethanol und rührt weiterhin 12 h. Man konzentriert im Vakuum, versetzt mit Wasser, extrahiert mit Dichlormethan und engt im Vakuum die mit Magnesiumsulfat getrocknete organische Schicht ein. Den Rückstand versetzt man mit 0,3 ml konzentrierter Salzsäure und Aceton und erhält 1,5 g der Titelverbindung. Schmp. 274-275°C (Zers.).

### 7. 3-[2-(2,3-Dimethyl-imidazo[1,2-a]pyridin-8-ylaminomethyl)-3-methylphenyl]-4-methyl-oxazolidin-2-on

a) Analog Beispiel 5b erhält man aus Chlorameisensäure-(2-chlorpropylester) und 8-(2-Amino-6-methyl-benzylamino)-2,3-dimethyl-imidazo-[1,2-a]pyridin 8-[2-(2-Chlorpropoxycarbonylamino)-6-methyl-benzylamino]-2,3-dimethyl-imidazo[1,2-a]pyridin.

b) Aus voranstehender Verbindung und einer Lösung von Kaliumhydroxid in Ethanol erhält man analog Beispiel 5c die Titelverbindung.

### 8. 3-[2-(2,3-Dimethyl-imidazo[1,2-a]pyridin-8-ylaminomethyl-3-methyl-phenyl]-5-methoxymethyl-oxazolidin-2-on

a) Analog Beispiel 5b erhält man 8-[2-(1-Chlormethyl-2-methoxyethoxycarbonylamino)-6-methyl-benzylamino]-2,3-dimethyl-imidazo[1,2-a]pyridin aus Chlorameisensäure-(1-chlormethyl-2-methoxyethylester) (Helv.Chim. Acta 44, 105, 1961) und 8-(2-Amino-6-methyl-benzylamino)-2,3-dimethylimidazo[1,2-a]pyridin.

b) Aus voranstehender Verbindung und einer Lösung von Kaliumhydroxid in Ethanol erhält man analog Beispiel 5c die Titelverbindung.

### 9. 1-[2-(2,3-Dimethyl-imidazo[1,2-a]pyridin-8-ylaminomethyl)-3-methylphenyl]-pyrrolidin-2-on

### a) 8-[2-(4-Chlorbutyrylamino)-6-methyl-benzylamino]-2,3-dimethyl-imidazo[1,2-a]pyridin

Man tropft zu einer Lösung von 2,2 g (7,85 mmol) 8-(2-Amino-6-methylbenzylamino)-2,3-dimethylimidazo[1,2-a]pyridin und 0,63 ml (7,85 mmol) Pyridin in 30 ml Dichlormethan bei 0-5°C 0,88 ml (7,85 mmol) 4-Chlorbutyrylchlorid unter Rühren zu und rührt noch 2 h bei 0-20°C. Man versetzt mit Wasser und Kaliumhydrogencarbonat bis pH 7, schüttelt aus, trocknet die organische Schicht mit Magnesiumsulfat und engt im Vakuum ein. Der Rückstand wird aus Toluol und Petrolether (50/70°C) umkristallisiert. Man erhält 2,6 g (86 %) der Titelverbindung. Schmp. 138-140°C.

### b) 1-[2-(2,3-Dimethyl-imidazo[1,2-a]pyridin-8-ylaminomethyl)-3-methylphenyl]-pyrrolidin-2-on

Zu einer Lösung von 2,4 g (6,24 mmol) der voranstehenden Verbindung in 60 ml Ethanol tropft man bei RT eine Lösung von 0,41 g (6,25 mmol) 85 % KOH in 15 ml Ethanol und rührt noch 4 h. Man konzentriert im Vakuum auf ca. 30 ml, verdünnt mit Wasser und schüttelt mehrmals mit Essigsäureethylester aus. Die organische Schicht wird mit Magnesiumsulfat getrocknet, im Vakuum eingeengt und der Rückstand mit Diisopropylether zur Kristallisation gebracht. Man erhält 2,0 g (92 %) der Titelverbindung. Schmp. 138-141°C.

### 10. 1-[2-(3-Chlor-2-methyl-imidazo[1,2-a]pyridin-8-yl-aminomethyl)-3-methyl-phenyl]-pyrolidin-2-on

### a) 8-[2-(4-Chlorbutyrylamino)-6-methyl-benzylamino]-3-chlor-2-methyl-imidazo[1,2-a]pyridin

Nach der in Beispiel 9a) angegebenen Arbeitsweise erhält man die Titelverbindung durch Umsetzung von 8-(2-Amino-6-methyl-benzylamino)-3-chlor-2-methyl-imidazo[1,2-a]pyridin und 4-Chlorbutyrylchlorid nach Kristallisation aus Essigester/Cyclohexan als beiges Pulver. Ausb. 66 %; Schmp. 127-130°C.

### b) 1-[2-(3-Chlor-2-methyl-imidazo[1,2-a]pyridin-8-yl-aminomethyl)-3-methyl-phenyl]-pyrrolidin-2-on

Nach der in Beispiel 9b) angegebenen Arbeitsweise erhält man die Titelverbindung durch Umsetzung der voranstehenden Verbindung mit Kaliumhydroxid in Ethanol und anschließender Kristallisation aus Diisopropylether als beiges Pulver. Ausb. 77 %; Schmp. 265-267°C.

### Ausgangsverbindungen

### A1. 3-Chlor-2-methyl-8-pivaloylamino-imidazo[1,2-a]pyridin

Man löst 5,0 g (18,6 mmol) 2-Methyl-8-pivaloylamino-imidazo[1,2-a]pyridinhydrochlorid, hergestellt aus 8-Amino-2-methyl-imidazo[1,2-a]pyridin und Pivaloylchlorid, Schmp. 229-230°C, in Eisessig (20 ml) und leitet bei 15°C langsam Chlorgas ein, bis die Reaktion nach DC-Kontrolle beendet ist (ca. 20 min). Dann wird das Lösungsmittel abdestilliert, der Rückstand in Essigester/Wasser (jeweils 30 ml) aufgenommen, mit gesättigter Natriumhydrogencarbonatlösung basisch gestellt und extrahiert. Anschließend wird nochmals mit Essigester (3 x 30 ml) extrahiert. Die vereinigten organischen Extrakte werden mit Wasser (50 ml) gewaschen, über Magnesiumsulfat getrocknet und eingeengt. Der Rückstand wird durch Chromatographie an Kieselgel (Fließmittel: Toluol/Dioxan = 9:1) gereinigt. Nach Einengen der Fraktionen mit Rf = 0,2 erhält man die Titelverbindung (4,1 g, 83 %) als farblosen Feststoff. Schmp. 117-118°C.

### A2. 8-Amino-3-chlor-imidazo[1,2-a]pyridin

Eine Suspension von 3-Chlor-2-methyl-8-pivaloylamino-imidazo[1,2-a]pyridin (4,0 g, 15 mmol) in 60 %iger Schwefelsäure (25 ml) wird 1 h bei 100°C gerührt. Nach Abkühlen auf RT wird Wasser (100 ml) zugegeben und mit 10 N Natronlauge auf pH 10 gestellt. Dann wird mit Essigester ( 3 x 50 ml) extrahiert. Die vereinigten organischen Extrakte werden mit Wasser (50 ml) gewaschen, über Magnesiumsulfat getrocknet und eingeengt. Der Rückstand wird in siedendem Toluol aufgenommen, mit Kieselgel geklärt und kristallisiert. Die Titelverbindung wird als beiger Feststoff isoliert. Ausbeute 1,9 g (70 %), Schmp. 126-127°C.

### B1. 8-(6-Methyl-2-nitro-benzylamino)-2,3-dimethyl-imidazo[1,2-a]pyridin

Zu einer Lösung von 8-Amino-2,3-dimethylimidazo[1,2-a]pyridin (14,7 g) und 6-Methyl-2-nitrobenzylchlorid (18,6 g) in 100 ml Aceton gibt man bei RT 15,0 g Natriumjodid und 31,0 g Natriumcarbonat und erhitzt anschließend für 6 h unter Rückfluß zum Sieden. Nach Abkühlen der Lösung auf RT und Einengen wird der Rückstand in einem Gemisch aus 200 ml Ethylacetat und 200 ml Wasser gelöst und die organische Phase wird abgetrennt. Nach drei weiteren Extraktionen mit jeweils 100 ml Ethylacetat werden die vereinigten organischen Phasen über Magnesiumsulfat getrocknet und anschließend auf 80 ml eingeengt. 12,1 g der Titelverbindung kristallisieren als schwach gelber Feststoff. Die Mutterlauge wird eingeengt. Nach chromatografischer Reinigung des Rückstandes an Kieselgel (Fließmittel: Toluol/Dioxan = 6:1) erhält man weitere 14 g des kristallinen Produkts. Nach Umkristallisation beider Fraktionen aus Ethylacetat erhält man 21,5 g (76 %) der Titelverbindung vom Schmp. 160-162°C.

### B2. 8-(6-Methyl-2-nitro-benzyloxy)-2,3-dimethyl-imidazo[1,2-a]pyridin

Eine Suspension von 8-Hydroxy-2,3-dimethyl-imidazo[1,2-a]pyridin (7,46 g, 46 mmol), 6-Methyl-2-nitrobenzylchlorid ( 9,35 g, 50,4 mmol), Natriumcarbonat (11,24 g, 105 mmol) und Natriumjodid (7,63 g, 50,4 mmol) in Aceton (750 ml) wird 8 h am Rückfluß erhitzt. Nach Abkühlen auf RT wird Wasser (200 ml) zugegeben und das Aceton am Rotationsverdampfer abdestilliert. Der wäßrige Rückstand wird anschließend 30 min bei 4°C gerührt. Danach wird der hellbraune Niederschlag abfiltriert, mit Wasser gewaschen und im Vakuum getrocknet. Weitere Reinigung erfolgt durch Chromatographie an Kieselgel (Fließmittel: Toluol/Dioxan = 5:1). Die Fraktionen mit Rf = 0,2 werden eingeengt und aus Essigester/Cyclohexan umkristallisiert. Die Titelverbindung wird als gelber Feststoff isoliert. Ausbeute 8,31 g (58 %), Schmp. 168-171°C.

### B3. 3-Chlor-2-methyl-8-(6-methyl-2-nitro-benzylamino)-imidazo[1,2-a]pyridin

Ausgehend von 8-Amino-3-chlor-imidazo[1,2-a]pyridin (9,26 g), 6-Methyl-2-nitrobenzylchlorid (10,5 g), Natriumcarbonat (13,7 g) und Natriumjodid (8,55 g) in Aceton (380 ml) erhält man nach der in Beispiel B1 angegebenen Arbeitsweise nach Chromatographie an Kieselgel (Fließmittel: Toluol/Dioxan = 20:1) und Kristallisation aus Essigester/Cyclohexan 10,6 g (63 %) der Titelverbindung vom Schmp. 142-144°C.

### B4. 8-(2-tert.-Butoxycarbonylamino-6-methyl-benzylamino)-2,3-dimethylimidazo[1,2-a]pyridin

Ausgehend von 8-Amino-2,3-dimethylimidazo[1,2-a]pyridin (4,8 g), 2-tert.-Butoxycarbonylamino-6-methylbenzylchlorid (9,2 g), Natriumjodid (5,5 g) und Natriumcarbonat (8,0 g) in Aceton (250 ml) erhält man in analoger Anwendung des Verfahrens von Beispiel B1 nach Chromatografie an Kieselgel (Fließmittel Toluol/Dioxan 20:1) und Umkristallisation aus Diisopropylether 7,1 g (62 %) der Titelverbindung vom Schmp. 149-152°C.

### B5. 8-(2-tert.-Butoxycarbonylamino-6-methyl-benzyloxy)-2,3-dimethyl-imidazo[1,2-a]pyridin

Ausgehend von 8-Hydroxy-2,3-dimethyl-imidazo[1,2-a]pyridin (1,6 g), 2-tert.-Butoxycarbonylamino-6-methylbenzylchlorid (3,1 g), Natriumjodid (1,8 g) und Natriumcarbonat (2,7 g) in Aceton (350 ml) erhält man in analoger Anwendung des Verfahrens von Beispiel B1 nach Chromatografie an Kieselgel (Fließmittel Toluol/Dioxan 5:1) und Umkristallisation aus Cyclohexan 3,0 g (78 %) der Titelverbindung vom Schmp. 128-131°C.

### B6. 8-(2-tert.-Butoxycarbonylamino-6-methyl-benzylamino)-3-formyl-2-methylimidazo[1,2-a]pyridin

Ausgehend von 8-Amino-3-formyl-2-methylimidazo[1,2-a]pyridin (4,0 g), 2-tert.-Butoxycarbonylamino-6-methylbenzylchlorid (7,0 g), Natriumjodid (4,1 g) und Natriumcarbonat (6,1 g) in Aceton (250 ml) erhält man in analoger Anwendung des Verfahrens von Beispiel B1 nach Chromatografie an Kieselgel (Fließmittel Toluol/Dioxan 9:1) und Umkristallisation aus Diisopropylether 7,3 g (81 %) der Titelverbindung vom Schmp. 210-212°C.

### B7. 8-(2-tert.-Butoxvcarbonylamino-6-methyl-benzyloxy)-3-formyl-2-methylimidazo[1,2-a]pyridin

Ausgehend von 3-Formyl-8-hydroxy-2-methylimidazo[1,2-a]pyridin (2,4 g), 2-tert.-Butoxycarbonylamino-6-methylbenzylchlorid (4,2 g), Natriumjodid (2,5 g) und Natriumcarbonat (3,7 g) in Aceton (400 ml) erhält man in analoger Anwendung des Verfahrens von Beispiel B1 nach Umkristallisation aus Diisopropylether/Ethylacetat 4,4 g (80 %) der Titelverbindung vom Schmp. 189-191°C.

### C1. 8-(2-Amino-6-methyl-benzylamino)-2,3-dimethyl-imidazo[1,2-a]pyridin

### Methode A:

Eine Lösung von 8-(6-Methyl-2-nitrobenzylamino)-2,3-dimethylimidazo[1,2-a]-pyridin (61 g) in Methanol (5,5 l) wird in Gegenwart von 15 g Palladium auf Aktivkohle (5 %) als Katalysator bei RT und unter Atmosphärendruck für 1,5 h hydriert. Nach Abfiltrieren des Katalysators und Einengen wird der Rückstand in siedendem Ethylacetat gelöst (2,7 l). Nach dem Abkühlen auf RT werden 51 g (82 %) der Titelverbindung vom Schmp. 206-208°C isoliert.

### Methode B:

6,7 g 8-(2-tert.-Butoxycarbonylamino-6-methylbenzylamino)-2,3-dimethylimidazo[1,2-a]pyridin werden bei 25-30°C portionsweise zu einer Mischung aus Trifluoressigsäure (30 ml) und Anisol (3 ml) zugefügt. Nach 30-minütigem Rühren bei RT wird die Lösung in 100 ml Eiswasser gegossen und anschließend mit 75 ml 6N Natronlauge versetzt. Der Niederschlag wird abfiltriert und an Kieselgel chromatografisch gereinigt (Lösungsmittel: Toluol/Dioxan = 8:1). Nach dem Umkristallisieren aus Ethylacetat erhält man 3,1 g (62 %) der Titelverbindung vom Schmp. 206-208°C.

### C2. 8-(2-Amino-6-methyl-benzylamino)-3-formyl-2-methyl-imidazo[1,2-a]pyridin

Ausgehend von 8-(2-tert.-Butoxycarbonylamino-6-methyl-benzylamino)-3-formyl-2-methyl-imidazo[1,2-a]pyridin (3,6 g), Trifluoressigsäure (15 ml) und Anisol (5 ml) erhält man nach der für Beispiel C1 (Methode B) beschriebenen Arbeitsweise nach Chromatographie an Kieselgel (Fließmittel: Toluol/Dioxan = 9:1) und Kristallisation aus Essigester/Cyclohexan 2,3 g (76 %) der Titelverbindung vom Schmp. 230-234°C.

### C3. 8-(2-Amino-6-methyl-benzyloxy)-3-formyl-2-methyl-imidazo[1,2-a]pyridin

Ausgehend von 8-(2-tert.-Butoxycarbonylamino-6-methylbenzyloxy)-3-formyl-2-methylimidazo[1,2-a]pyridin (5,0 g) und Trifluoressigsäure (40 ml) erhält man in analoger Anwendung des Verfahrens von Beispiel C1 (Methode B) 3,57 g (96 %) der Titelverbindung vom Schmp. 144-150°C (Zers.).

### C4. 8-(2-Amino-6-methyl-benzylamino)-3-chlor-2-methyl-imidazo[1,2-a]pyridin-hydrochlorid

Eine Lösung von 8-(2-Nitro-6-methyl-benzylamino)-3-chlor-2-methyl-imidazo[1,2-a]pyridin (2,0 g, 6 mmol) in Methanol (175 ml) und Dioxan (175 ml) wird mit Platin-auf-Kohle-Katalysator (5 %ig) versetzt und 2 h bei RT und Atmosphärendruck hydriert. Nach 2 h wird 2 N Salzsäure (5 ml) zugegeben und nochmals 1 h unter denselben Bedingungen hydriert. Danach wird der Katalysator abfiltriert, das Filtrat mit 2 N Natronlauge auf pH 8,5 gestellt und das Lösungsmittel am Rotationsverdampfer abdestilliert. Der Rückstand wird in siedendem Essigester (400 ml) gelöst. Nach Abkühlen auf RT wird Diisopropylether (250 ml) zugegeben und zur Vervollständigung der Kristallisation 30 min bei 4°C gerührt. Danach wird der Niederschlag abgesaugt, mit Diisopropylether gewaschen und im Vakuum getrocknet. Die Titelverbindung (1,66 g, 92 %) wird als beiger Feststoff isoliert. Schmp. 243-246°C.

### Gewerbliche Anwendbarkeit

Die Verbindungen der Formel I und ihre Salze besitzen wertvolle pharmakologische Eigenschaften, die sie gewerblich verwertbar machen. Sie weisen insbesondere eine ausgeprägte Magensäuresekretionshemmung und eine ausgezeichnete Magen- und Darmschutzwirkuny bei Warmblütern auf. Hierbei zeichnen sich die erfindungsgemäßen Verbindungen durch eine hohe Wirkungsselektivität, eine vergleichsweise lange Wirkungsdauer, eine gute enterale Wirksamkeit, das Fehlen wesentlicher Nebenwirkungen und eine große therapeutische Breite aus.

Unter "Magen- und Darmschutz" wird in diesem Zusammenhang die Verhütung und Behandlung gastrointestinaler Krankheiten, insbesondere gastrointestinaler entzündlicher Krankheiten und Läsionen (wie z.B. Ulcus ventriculi, Ulcus duodeni, Gastritis, hyperazider oder medikamentös bedingter Reizmagen) verstanden, die beispielsweise durch Mikroorganismen (z.B. Helicobacter pylori), Bakterientoxine, Medikamente (z.B. bestimmte Antiphlogistika und Antirheumatika), Chemikalien (z.B. Ethanol), Magensäure oder Streßsituationen verursacht werden können. Die erfindungsgemäßen Verbindungen besitzen hierbei auch eine Eigenwirkung gegen den Keim Helicobacter pylori.

In ihren ausgezeichneten Eigenschaften erweisen sich die erfindungsgemäßen Verbindungen an verschiedenen Modellen, in denen die antiulcerogenen und die antisekretorischen Eigenschaften bestimmt werden, überraschenderweise den aus dem Stand der Technik bekannten Verbindungen deutlich überlegen. Aufgrund dieser Eigenschaften sind die Verbindungen der Formel I und ihre pharmakologisch verträglichen Salze für den Einsatz in der Human- und Veterinärmedizin hervorragend geeignet, wobei sie insbesondere zur Behandlung und/oder Prophylaxe von Erkrankungen des Magens und/oder Darms verwendet werden.

Ein weiterer Gegenstand der Erfindung sind daher die erfindungsgemäßen Verbindungen zur Anwendung bei der Behandlung und/oder Prophylaxe der vorstehend genannten Krankheiten.

Ebenso umfaßt die Erfindung die Verwendung der erfindungsgemäßen Verbindungen zur Herstellung von Arzneimitteln, die zur Behandlung und/oder Prophylaxe der vorstehend genannten Krankheiten eingesetzt werden.

Weiterhin umfaßt die Erfindung die Verwendung der erfindungsgemäßen Verbindungen zur Behandlung und/oder Prophylaxe der vorstehend genannten Krankheiten.

Ein weiterer Gegenstand der Erfindung sind Arzneimittel, die ein oder mehrere Verbindungen der Formel I und/oder ihre pharmakologisch verträglichen Salze enthalten.

Die Arzneimittel werden nach an sich bekannten, dem Fachmann geläufigen Verfahren hergestellt. Als Arzneimittel werden die erfindungsgemäßen pharmakologisch wirksamen Verbindungen (= Wirkstoffe) entweder als solche, oder vorzugsweise in Kombination mit geeigneten pharmazeutischen Hilfs- oder Trägerstoffen in Form von Tabletten, Dragees, Kapseln, Suppositorien, Pflastern (z.B. als TTS), Emulsionen, Suspensionen oder Lösungen eingesetzt, wobei der Wirkstoffgehalt vorteilhafterweise zwischen 0,1 und 95 % beträgt und wobei durch die entsprechende Wahl der Hilfs- und Trägerstoffe eine auf den Wirkstoff und/oder auf den gewünschten Wirkungseintritt genau angepaßte galenische Darreichungsform (z.B. eine Retardform oder eine magensaftresistente Form) erzielt werden kann.

Welche Hilfs- bzw. Trägerstoffe für die gewünschten Arzneimittelformulierungen geeignet sind, ist dem Fachmann aufgrund seines Fachwissens geläufig. Neben Lösemitteln, Gelbildnern, Suppositoriengrundlagen, Tablettenhilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler, Farbstoffe oder insbesondere Permeationspromotoren und Komplexbildner (z.B. Cyclodextrine) verwendet werden.

Die Wirkstoffe können oral, parenteral oder percutan appliziert werden.

Im allgemeinen hat es sich in der Humanmedizin als vorteilhaft erwiesen, den oder die Wirkstoffe bei oraler Gabe in einer Tagesdosis von etwa 0,01 bis etwa 20, vorzugsweise 0,05 bis 5, insbesondere 0,1 bis 1,5 mg/kg Körpergewicht, gegebenenfalls in Form mehrerer, vorzugsweise 1 bis 4 Einzelgaben zur Erzielung des gewünschten Ergebnisses zu verabreichen. Bei einer parenteralen Behandlung können ähnliche bzw. (insbesondere bei der intravenösen Verabreichung der Wirkstoffe) in der Regel niedrigere Dosierungen zur Anwendung kommen. Die Festlegung der jeweils erforderlichen optimalen Dosierung und Applikationsart der Wirkstoffe kann durch jeden Fachmann aufgrund seines Fachwissens leicht erfolgen.

Sollen die erfindungsgemäßen Verbindungen und/oder Salze zur Behandlung der oben genannten Krankheiten eingesetzt werden, so können die pharmazeutischen Zubereitungen auch einen oder mehrere pharmakologisch aktive Bestandteile anderer Arzneimittelgruppen, wie Antacida, beispielsweise Aluminiumhydroxyd, Magnesiumaluminat; Tranquilizer, wie Benzodiazepine, beispielsweise Diazepam; Spasmolytika, wie z.B. Bietamiverin, Camylofin, Anticholinergica, wie z.B. Oxyphencyclimin, Phencarbamid; Lokalanaesthetika, wie z.B. Tetracain, Procain; gegebenenfalls auch Fermente, Vitamine oder Aminosäuren enthalten.

Hervorzuheben ist in diesem Zusammenhang insbesondere die Kombination der erfindungsgemäßen Verbindungen mit Pharmaka, die die Säuresekretion hemmen, wie beispielsweise H₂-Blockern (z.B. Cimetidin, Ranitidin), H⁺/K⁺-ATPase-Hemmstoffen (z.B. Omeprazol, Pantoprazol), oder ferner mit sogenannten peripheren Anticholinergika (z.B. Pirenzepin, Telenzepin) sowie mit Gastrin-Antagonisten mit dem Ziel, die Hauptwirkung in additivem oder überadditivem Sinn zu verstärken und/oder die Nebenwirkungen zu eliminieren oder zu verringern, oder ferner die Kombination mit antibakteriell wirksamen Substanzen (wie z.B. Cephalosporinen, Tetracyclinen, Nalidixinsäure, Penicillinen oder auch Wismutsalzen) zur Bekämpfung von Helicobacter pylori.

### Pharmakologie

Die ausgezeichnete Magenschutzwirkung und die magensäuresekretionshemmende Wirkung der erfindungsgemäßen Verbindungen kann in Untersuchungen an tierexperimentellen Modellen nachgewiesen werden. Die in dem nachstehend aufgeführten Modell untersuchten erfindungsgemäßen Verbindungen sind mit Nummern versehen worden, die den Nummern dieser Verbindungen in den Beispielen entsprechen.

### Prüfung der sekretionshemmenden Wirkung am perfundierten Rattenmagen

In der folgenden Tabelle A ist der Einfluß der erfindungsgemäßen Verbindungen nach intravenöser Gabe auf die durch Pentagastrin stimulierte Säuresekretion des perfundierten Rattenmagens in vivo dargestellt.

**Tabelle A**

| Nr. | Dosis (µmol/kg) i.v. | Hemmung der Säureausscheidung (%) |
|---|---|---|
| 1b | 3 | 100 |
| 9b | 3 | 100 |

### Methodik

Narkotisierten Ratten (CD-Ratte, weiblich, 200-250 g; 1,5 g/kg i.m. Urethan) wurde nach Tracheotomie das Abdomen durch einen medianen Oberbauchschnitt eröffnet und ein PVC-Katheter transoral im Ösophagus sowie ein weiterer via Pylorus derart fixiert, daß die Schlauchenden eben noch in das Magenlumen hineinragten. Der aus dem Pylorus führende Katheter führte über eine seitliche Öffnung in der rechten Bauchwand nach außen.

Nach gründlicher Spülung (ca. 50-100 ml) wurde der Magen mit 37°C warmer physiologischer NaCl-Lösung kontinuierlich durchströmt (0,5 ml/min, pH 6,8-6,9; Braun-Unita I). In dem jeweils im 15 Minutenabstand aufgefangenen Effluat wurde der pH-Wert (pH-Meter 632, Glaselektrode EA 147; φ = 5 mm, Metrohm) sowie durch Titration mit einer frisch zubereiteten 0,01 N NaOH bis pH 7 (Dosimat 665 Metrohm) die sezernierte HCl bestimmt.

Die Stimulation der Magensekretion erfolgte durch Dauerinfusion von 1 µg/kg (= 1,65 ml/h) i.v. Pentagastrin (V. fem. sin.) ca. 30 min nach Operationsende (d.h. nach Bestimmung von 2 Vorfraktionen). Die zu prüfenden Substanzen wurden intravenös in 1 ml/kg Flüssigkeitsvolumen 60 min nach Beginn der Pentagastrin-Dauerinfusion verabreicht.

Die Körpertemperatur der Tiere wurde durch Infrarot-Bestrahlung und Heizkissen (automatische, stufenlose Regelung über rektalen Temperaturfühler) auf konstant 31,8-38°C gehalten.

In der Tabelle ist diejenige Dosis angegeben, die zu einer maximalen Hemmung der Säuresekretion um ca. 100 % führte.

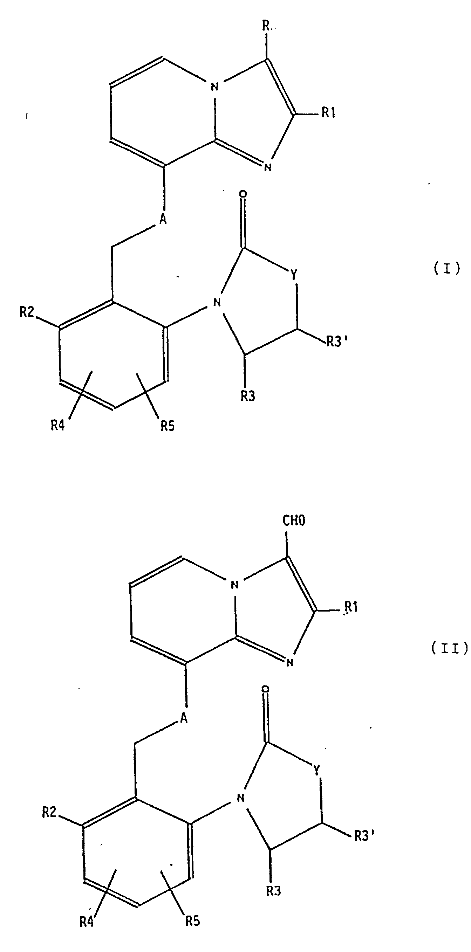

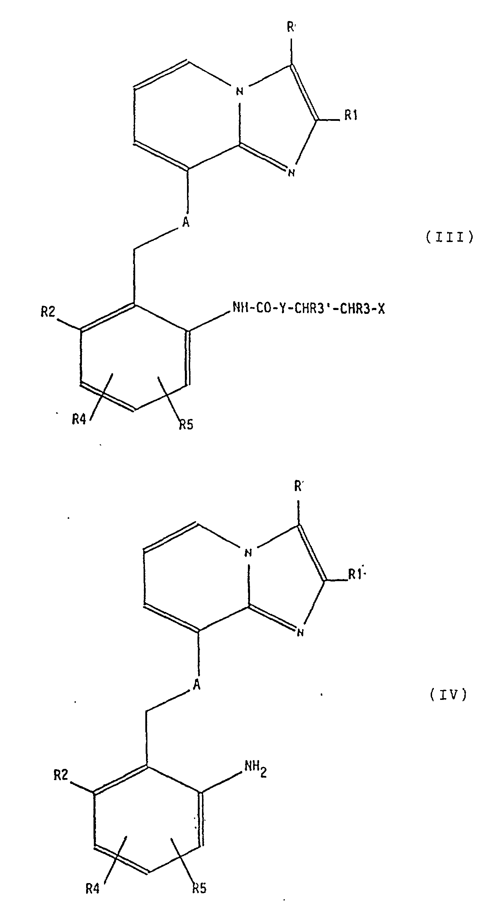

## Patentansprüche

1. Verbindungen der Formel I, 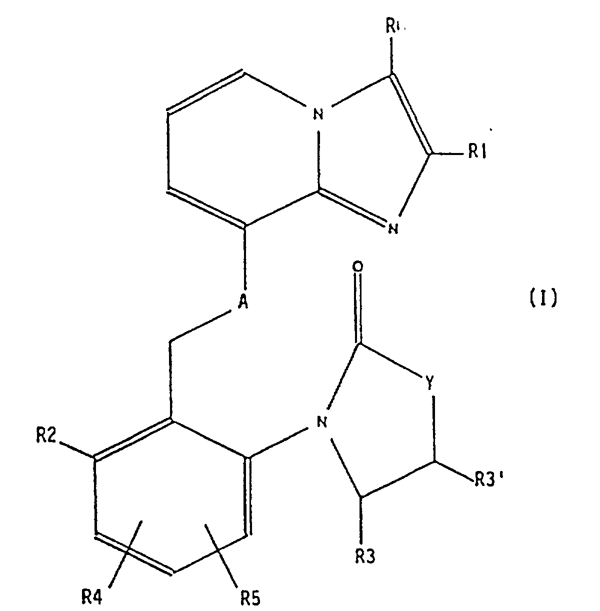 worin
R 1-4C-Alkyl, Hydroxymethyl, Halogen oder Thiocyanat,
R1 1-4C-Alkyl,
R2 Wasserstoff, 1-4C-Alkyl, 1-4C-Alkoxy, Halogen oder Trifluormethyl,
R3 Wasserstoff oder 1-4C-Alkyl,
R3' Wasserstoff, 1-4C-Alkyl oder substituiertes 1-4C-Alkyl mit einem oder zwei gleichen oder verschiedenen Substituenten ausgewählt aus der Gruppe bestehend aus Halogen, 1-4C-Alkoxy oder 1-4C-Alkoxy-1-4C-alkoxy,
R4 Wasserstoff, 1-4C-Alkyl, 1-4C-Alkoxy, Halogen oder Trifluormethyl,
R5 Wasserstoff, 1-4C-Alkyl, 1-4C-Alkoxy oder Halogen,
A 0 (Sauerstoff) oder NH und
Y 0 (Sauerstoff) oder CH₂ bedeutet,
und ihre Salze.

2. Verbindungen der Formel I nach Anspruch 1, worin
R 1-4C-Alkyl, Hydroxymethyl oder Halogen,
R1 1-4C-Alkyl,
R2 1-4C-Alkyl oder Halogen,
R3 Wasserstoff oder 1-4C-Alkyl,
R3' Wasserstoff, 1-4C-Alkyl oder substituiertes 1-4C-Alkyl mit einem Substituenten ausgewählt aus der Gruppe bestehend aus Halogen, 1-4C-Alkoxy und 1-4C-Alkoxy-1-4C-alkoxy,
R4 Wasserstoff,
R5 Wasserstoff,
A 0 (Sauerstoff) oder NH und
Y 0 (Sauerstoff) oder CH₂ bedeutet,
und ihre Salze.

3. Verbindungen der Formel I nach Anspruch 1, worin
R Methyl, Hydroxymethyl,Chlor oder Fluor,
R1 Methyl,
R2 1-4C-Alkyl,
R3 Wasserstoff oder 1-4C-Alkyl,
R3' Wasserstoff, 1-4C-Alkyl oder substituiertes 1-4C-Alkyl mit einem Substituenten ausgewählt aus der Gruppe bestehend aus 1-4C-Alkoxy und 1-4C-Alkoxy-1-4C-alkoxy,
R4 Wasserstoff,
R5 Wasserstoff,
A 0 (Sauerstoff) oder NH und
Y 0 (Sauerstoff) oder CH₂ bedeutet,
und ihre Salze.

4. Verbindungen der Formel I nach Anspruch 1, worin
R Methyl, Hydroxymethyl oder Chlor,
R1 Methyl,
R2 1-4C-Alkyl,
R3 Wasserstoff oder 1-4C-Alkyl,
R3' Wasserstoff, 1-4C-Alkyl, 1-4C-Alkoxy-1-4C-alkyl oder 1-4C-Alkoxy-1-4C-alkoxy-1-4C-alkyl,
R4 Wasserstoff,
R5 Wasserstoff,
A 0 (Sauerstoff) oder NH
Y 0 (Sauerstoff) oder CH₂ bedeutet,
und ihre Salze.

5. Verbindungen der Formel I nach Anspruch 1, worin R Fluor bedeutet.

6. Arzneimittel enthaltend eine Verbindung nach Anspruch 1 und/oder ein pharmakologisch verträgliches Salz davon zusammen mit üblichen pharmazeutischen Hilfs- und/oder Trägerstoffen.

7. Verbindungen nach Anspruch 1 und ihre pharmakologisch verträglichen Salze zur Anwendung bei der Verhütung und Behandlung gastrointestinaler Krankheiten.

8. Verwendung von Verbindungen nach Anspruch 1 und ihren pharmakologisch verträglichen Salzen zur Herstellung von Arzneimitteln für die Verhütung und Behandlung gastrointestinaler Krankheiten.

## Claims

1. Compounds of the formula I 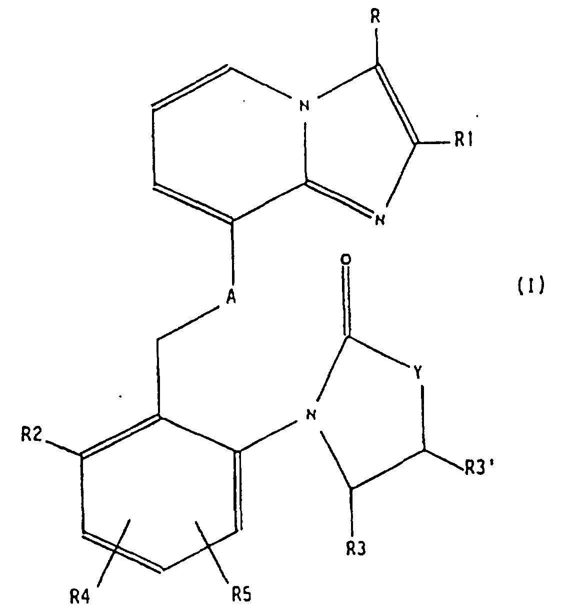 in which
R is 1-4C-alkyl, hydroxymethyl, halogen or thiocyanate,
R1 is 1-4C-alkyl,
R2 is hydrogen, 1-4C-alkyl, 1-4C-alkoxy, halogen or trifloromethyl,
R3 is hydrogen or 1-4C-alkyl,
R3' is hydrogen, 1-4C-alkyl or substituted 1-4C-alkyl having one or two identical or different substituents selected from the group consisting of halogen, 1-4C-alkoxy and 1-4C-alkoxy-1-4C-alkoxy,
R4 is hydrogen, 1-4C-alkyl, 1-4C-alkoxy, halogen or trifluoromethyl,
R5 is hydrogen, 1-4C-alkyl, 1-4C-alkoxy or halogen,
A is O (oxygen) or NH and
Y is O (oxygen) or CH₂,
and their salts.

2. Compounds of the formula I as claimed in claim 1, in which
R is 1-4C-alkyl, hydroxymethyl or halogen,
R1 is 1-4C-alkyl,
R2 is 1-4C-alkyl or halogen,
R3 is hydrogen or 1-4C-alkyl,
R3' is hydrogen, 1-4C-alkyl or substituted 1-4C-alkyl having a substituent selected from the group consisting of halogen, 1-4C-alkoxy and 1-4C-alkoxy-1-4C-alkoxy,
R4 is hydrogen,
R5 is hydrogen,
A is O (oxygen) or NH and
Y is O (oxygen) or CH₂,
and their salts.

3. Compounds of the formula I as claimed in claim 1, in which
R is methyl, hydroxymethyl, chlorine or fluorine,
R1 is methyl,
R2 is 1-4C-alkyl,
R3 is hydrogen or 1-4C-alkyl,
R3' is hydrogen, 1-4C-alkyl or substituted 1-4C-alkyl having a substituent selected from the group consisting of 1-4C-alkoxy and 1-4C-alkoxy-1-4C-alkoxy,
R4 is hydrogen,
R5 is hydrogen,
A is O (oxygen) or NH and
Y is O (oxygen) or CH₂.
and their salts.

4. Compounds of the formula I as claimed in claim 1, in which
R is methyl, hydroxymethyl or chlorine,
R1 is methyl,
R2 is 1-4C-alkyl,
R3 is hydrogen or 1-4C-alkyl,
R3' is hydrogen, 1-4C-alkyl, 1-4C-alkoxy-1-4C-alkyl or 1-4C-alkoxy-1-4C-alkoxy-1-4C-alkyl,
R4 is hydrogen,
R5 is hydrogen,
A is O (oxygen) or NH
Y is O (oxygen) or CH₂,
and their salts.

5. Compounds of the formula I as claimed in claim 1, in which R is fluorine.

6. A medicament comprising a compound as claimed in claim 1 and/or a pharmacologically tolerable salt thereof together with customary pharmaceutical auxiliaries and/or excipients.

7. Compounds as claimed in claim 1 and their pharmacologically tolerable salts for use in the prevention and treatment of gastrointestinal diseases.

8. The use of compounds as claimed in claim 1 and their pharmacologically tolerable salts for the production of medicaments for the prevention and treatment of gastrointestinal diseases.

## Revendications

1. Composés de formule (I) 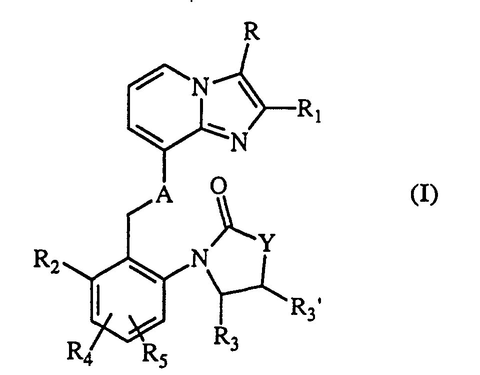 dans laquelle
R représente un groupe alkyle en C₁₋₄, hydroxyméthyle, un atome d'halogène ou un groupe thiocyanate,
R₁ représente un groupe alkyle en C₁₋₄,
R₂ représente un atome d'hydrogène, un groupe alkyle en C₁₋₄, alcoxy en C₁₋₄, un atome d'halogène ou un groupe trifluorométhyle,
R₃ représente un atome d'hydrogène ou un groupe alkyle en C₁₋₄,
R₃' représente un atome d'hydrogène, un groupe alkyle en C₁₋₄, ou alkyle en C₁₋₄ portant un ou deux substituants, identiques ou différents, choisis parmi les atomes d'halogène, les groupes alcoxy en C₁₋₄ ou (alcoxy en C₁₋₄)-(alcoxy en C₁₋₄),
R₄ représente un atome d'hydrogène, un groupe alkyle en C₁₋₄, alcoxy en C₁₋₄, halogéno ou trifluorométhyle,
R₅ représente un atome d'hydrogène, un groupe alkyle en C₁₋₄ ou alcoxy en C₁₋₄ ou un atome d'halogène,
A représente un atome d'oxygène ou un groupe NH, et
Y représente un atome d'oxygène ou un groupe CH₂,
et les sels de ces composés.

2. Composés de formule (I) selon la revendication 1, dans lesquels
R représente un groupe alkyle en C₁₋₄, hydroxyméthyle ou un atome d'halogène,
R₁ représente un groupe alkyle en C₁₋₄,
R₂ représente un groupe alkyle en C₁₋₄ ou un atome d'halogène,
R₃ représente un atome d'hydrogène ou un groupe alkyle en C₁₋₄,
R₃' représente un atome d'hydrogène, un groupe alkyle en C₁₋₄ ou alkyle en C₁₋₄ portant un substituant choisi parmi les atomes d'halogène, les groupes alcoxy en C₁₋₄ et (alcoxy en C₁₋₄)-(alcoxy en C₁₋₄),
R₄ représente un atome d'hydrogène,
R₅ représente un atome d'hydrogène,
A représente un atome d'oxygène ou un groupe NH, et
Y représente un atome d'oxygène ou un groupe CH₂,
et les sels de ces composés.

3. Composés de formule (I) selon la revendication 1, dans lesquels
R représente un groupe méthyle, hydroxyméthyle ou un atome de chlore ou de fluor,
R₁ représente un groupe méthyle,
R₂ représente un groupe alkyle en C₁₋₄,
R₃ représente un atome d'hydrogène ou un groupe alkyle en C₁₋₄,
R₃' représente un atome d'hydrogène, un groupe alkyle en C₁₋₄ ou alkyle en C₁₋₄ portant un substituant choisi parmi les groupes alcoxy en C₁₋₄ et (alcoxy en C₁₋₄)-(alcoxy en C₁₋₄),
R₄ représente un atome d'hydrogène,
R₅ représente un atome d'hydrogène,
A représente un atome d'oxygène ou un groupe NH, et
Y représente un atome d'oxygène ou un groupe CH₂,
et les sels de ces composés.

4. Composés de formule (I) selon la revendication 1, dans lesquels
R représente un groupe méthyle, hydroxyméthyle ou un atome de chlore,
R₁ représente un groupe méthyle,
R₂ représente un groupe alkyle en C₁₋₄,
R₃ représente un atome d'hydrogène ou un groupe alkyle en C₁₋₄,
R₃' représente un atome d'hydrogène, un groupe alkyle en C₁₋₄, (alcoxy en C₁₋₄)-(alkyle en C₁₋₄) ou (alcoxy en C₁₋₄)-(alcoxy en C₁₋₄)-(alkyle en C₁₋₄),
R₄ représente un atome d'hydrogène,
R₅ représente un atome d'hydrogène,
A représente un atome d'oxygène ou un groupe NH, et
Y représente un atome d'oxygène ou un groupe CH₂,
et les sels de ces composés.

5. Composés de formule (I) selon la revendication 1, dans lesquels R représente un atome de fluor.

6. Médicaments contenant un composé selon la revendication 1 et /ou un sel pharmacologiquement acceptable d'un tel composé, en combinaison avec des additifs et/ou excipients pharmaceutiques usuels.

7. Composés selon la revendication 1 et leur sels pharmacologiquement acceptables, destinés à être utilisés pour la prévention et le traitement de maladies gastrointestinales.

8. Utilisation de composés selon la revendication 1 et de leurs sels pharmacologiquement acceptables pour la fabrication de médicaments destinés à la prévention et au traitement de maladies gastrointestinales.
